# EUROPÄISCHE PATENTSCHRIFT

(11) **EP 1 651 134 B1**
(45) Veröffentlichungstag und Bekanntmachung des Hinweises auf die Patenterteilung: **23.07.2008**
(21) Anmeldenummer: 04763437.3
(22) Anmeldetag: 23.07.2004
(51) Int. Cl.: A61F 2/00, A61F 5/445

(54) **VERSCHLUSSSYSTEM FÜR EINEN NATÜRLICHEN ODER KÜNSTLICHEN DARMAUSGANG**
CLOSING SYSTEM FOR A NATURAL OR AN ARTIFICIAL ANUS
SYSTEME D'OBTURATION POUR ANUS NATUREL OU ARTIFICIEL

(30) Priorität: 23.07.2003 DE 10333706
(43) Veröffentlichungstag der Anmeldung: 03.05.2006
(73) Patentinhaber: Microcuff GmbH, 69469 Weinheim (DE)
(72) Erfinder: GÖBEL, Lothar, 97070 Würzburg (DE)
(74) Vertreter: Küchler, Stefan
(86) Internationale Anmeldenummer: PCT/EP2004/008256
(87) Internationale Veröffentlichungsnummer: WO 2005/009292

(56) Entgegenhaltungen:
- US-A- 2 324 520
- US-A- 3 802 418
- US-A- 3 828 782

## Beschreibung

Die Erfindung richtet sich auf ein Verschlußsystem für einen natürlichen oder künstlichen Darmausgang.

Die medizinische Versorgung eines Anus praeter ist nach wie vor ein aktuelles, tägliches Problem. Eine sehr verbreitete Methode ist das Auffangen des Stuhls in Gefäßen, die in Form von Klebebeuteln benutzt werden. Diese extrakorporale Speicherung ist mit der Problematik der Geruchsbelästigung, der Schmutzbelästigung und der Auslaufgefahr verbunden.

Neben den extrakorporalen Auffangsystemen wurden Verschlüsse entwickelt mit dem Ziel einer intrakorporalen Speicherung und späteren gezielten Entleerung des Stuhls. Diese Auffangsysteme haben wegen ihrer schwierigen Handhabung keine besondere Verbreitung gefunden. Die Problematik bestand darin, dass eine wirkliche Abdichtung nicht erreicht werden konnte. Die Verschlüsse waren dem variablen Bauchinnendruck nicht gewachsen.

Ähnliche Probleme haben unter Stuhlinkontinenz leidende Personen. Hier gilt es, den After mit einer geeigneten Vorrichtung zu verschließen, um eine unkontrollierte Darmentleerung zu vermeiden. Andererseits soll eine gewollte Entleerung begünstigt bzw. erleichtert werden. Hierfür verwendete Vorrichtungen umfassen stets ein in den After einzuführendes Rohr, das infolge seiner geringen Nachgiebigkeit fast immer Schmerzen verursacht und sogar Verletzungen mit sich bringen kann.

Aus diesen Nachteilen des beschriebenen Standes der Technik resultiert das die Erfindung initiierende Problem, ein Verschlußsystem für einen natürlichen oder künstlichen Darmausgang zu schaffen, das unkompliziert im Aufbau ist, leicht eingesetzt werden kann, einfach zu handhaben ist und eine möglichst geringe Druckbelastung des Gewebes, insbesondere der Darmschleimhaut, ergibt. Schließlich soll das System preisgünstig sein.

Die Lösung dieses Problems gelingt durch einen aufblasbaren Ballon mit einer etwa torusförmigen Struktur, wie er auch aus der Patentschrift US 2,324,520 bekannt ist. Der erfindungsgemäße Ballon wird allerdings aus einem flächigen, in sich umgestülpten Schlauchabschnitt gebildet, dessen beide Enden etwa koaxial ineinander verlaufen und mit (je) einer Hülse verbunden sind. Vorteilhafte Weiterbildungen der Erfindung sind in den Unteransprüchen enthalten.

Der Ballon wird präformiert hergestellt und muß daher nicht mit starkem Überdruck aufgeblasen werden, sondern nur mit einigen mbar Überdruck gegenüber dem ambienten Druck. Dadurch bleibt er auch in aufgeblasenem Zustand flexibel und kann sich den natürlichen Gegebenheiten anpassen, bspw. einer unvermittelten Umbiegung eines Darms folgen, etc. Der Druck auf die Darmschleimhaut ist stets etwa konstant und entspricht nur dem Innendruck des Ballons. Von großer Bedeutung ist, dass der Ballon keines führenden Schaftes bedarf, so dass selbst ein solches Element verringerten Durchmessers nicht in den Darm hineinragt. In Ermangelung eines führenden Schaftes wird bei der Erfindung die innere Wandung des Torus durch den Ballon selbst gebildet, woraus dessen hohe Flexibilität resultiert.

Die beiden Enden des Ballons befinden sich an ein und der selben Seite des Torus (bedingt durch die Umstülpung des Schlauchs), und zwar an derjenigen Seite, welche dem Körperinneren der Person abgewandt ist. Dort ist der Schlauch an einer oder mehreren Hülsen befestigt, welche sich nicht durch den Ballon hindurcherstrecken und kürzer sind als der aufgeblasene Ballon bzw. kürzer als die halbe Länge des ursprünglichen, noch nicht umgestülpten Schlauchs, vorzugsweise kleiner als ein Viertel dieser ursprünglichen (gesamten) Schlauchlänge, insbesondere kleiner als ein Sechstel dieser Länge.

Diese Hülsen befinden sich während der Anwendung vorzugsweise außerhalb des Körpers einer Person oder ragen nur geringfügig durch die Bauchdecke hindurch bzw. erstrecken sich gerade eben bis zum Schließmuskel. Dadurch kann der Tragekomfort erheblich gesteigert werden, weil der After bzw. das Stoma nicht permanent gedehnt wird. Durch die zueinander konzentrischen Enden des Ballons bzw. Anschlußstutzen wird durch die Präformierung ein gegenüber dem eigentlichen Ballon verjüngter Halsbereich geschaffen, welcher sich bspw. durch den After hindurch bis zu der Ampulla recti erstreckt, wo der torusförmig erweiterte Ballon Platz findet, um sich zu entfalten und sich dabei zu verankern.

Der Halsbereich selbst bleibt dank seines niedrigen Innendrucks flexibel und läßt sich querschnittlich zusammendrücken. Da ein Schlauchende enger ist als das andere, ist eine koaxiale Anordnung des zu dem eigentlichen Ballon führenden Halsbereichs nach erfolgter Umstülpung vorprogrammiert, es verbleibt sogar ein ringförmiger Zwischenraum, welcher eine Strömungsverbindung von dem torusförmigen Balloninnenraum zu einem hülsenseitigen Anschluß bildet.

Die durch die Präformierung hervorgerufene Trompetenform am vorderen Ende des aufgeblähten Ballons erleichtert ggf. das Hindurchleiten von Flüssigkeiten, des Stuhls, etc.

Andererseits läßt sich durch das zentrale Lumen, das nicht mit dem Innenraum des Ballons kommuniziert und daher von Druck völlig frei ist, zum Einführen von Rohren oder Schläuchen (Drainage) und/oder von Kathetern od. dgl. verwenden. Hierbei ist von Vorteil, dass das zentrale und druckfreie Innenlumen von dem Druck innerhalb des Torus flach gedrückt wird, so dass hier zwei Lagen des Schlauchs aneinanderliegen, wenn der aufgedehnte Ballonabschnitt des einwandigen, nicht invaginierten und übergewälzten Ausgangsschlauches, so gewählt wurde, dass seine Länge größer ist als sein Durchmesser. Der zusammengedrückte Innenschlauch des Doppelschlauchsegments übt dann einen Anpreßdruck auf einen eingeführten Gegenstand aus und hält diesen daher reibschlüssig fest.

Darüber hinaus werfen diese aneinanderliegenden Schlauchlagen in dem jeweiligen Randbereich zwei Falten mit endlichem Radius, wo demnach - bei freiem zentralen Lumen, d.h., ohne eingeführten Gegenstand - zwei enge kapillarenförmige Durchgangspassagen verbleiben, so dass sich bspw. ein erhöhter Innendruck im Darm auf natürlichem Weg abbauen kann.

Indem eine erfindungsgemäße Vorrichtung nur zum Teil in den natürlichen After eingeführt wird, läßt sich ein gegen den Schließmuskel arbeitender Innendruck erzeugen, der diesen zu einer Reaktion auffordert und dabei trainiert. Ein solches Training kann durch abwechselndes Aufpumpen und Ablassen des Ballons intensiviert werden.

In anderen Fällen kann das zentrale Lumen durch einen kurzes, bevorzugt dauerhaft fixiertes, eingestecktes Ringsegment offengehalten werden; solchenfalls empfiehlt sich die Verwendung eines in diesem zentralen Lumen nach diesem Ring angeordneten Verschlußelements, insbesondere getrennt aufblasbaren Ballons.

Der Ballon aus einem dünnwandigen, flexiblen und inflatierbaren Polymer wird in seinen äußeren Abmessungen im aufgeblasenen Zustand vorgefertigt. Ein Aufblasen des Ballons erfolgt nur zum Zweck der Entfaltung der Ballonhülle. Das für den Ballon verwendete Material läßt ein Ausdehnen des Ballons nur in sehr geringem Umfang zu, da es weitgehend unelastisch ist.

Als Polymer wird bevorzugt Polyurethan, eine Polyurethan-Polyvinylfluorid-Mischung oder ein vergleichbares Material auf Polyurethan-Basis verwendet. Dieses Material ist neutral, so dass es keinerlei schädliche Auswirkungen auf die Schleimhaut des Darmes haben kann.

Der Ballon ist in seiner einfachsten Ausführungsform mit einem Anschlußschlauchstutzen versehen, der mit dem Stopfen verbunden ist. Nach Einführung des Stopfens in die Bauchwand wird der Ballon durch einen im Stopfen befindlichen Kanal aufgefaltet und kommt mit seiner Außenwandung an der Darmwand zur Anlage. Um die Einführung des Ballons durch die Bauchwand in den Darm zu erleichtern, ist der Stopfen mit einem Hohlraum versehen, in dem der zusammengefaltete Ballon untergebracht werden kann.

Der Stopfen selbst wird bevorzugt formschlüssig mit einer an sich bekannten Verschlußkappe verbunden, die nach Einführung des Stopfens in die Bauchwand mit der Bauchwand verklebt werden kann.

An den Kanal des Stopfens kann zum Auffangen des Stuhlgangs ein Auffangbeutel angeschlossen werden.

Die bevorzugte Ausführungsform des Erfindungsgegenstands sieht jedoch vor, dass der Stopfen aus zwei ineinander steckbaren Hülsen besteht und dass der Ballon zwei Anschlußschlauchstutzen hat, deren Mündungen jeweils an eine der Hülsen angeschlossen sind. Dabei ist es günstig, wenn die eine Mündung einen an die Außenhülse angepaßten und die andere Mündung eine an die Innenhülse angepaßten Durchmesser hat. Beide Mündungen können mit den Hülsenwänden verklebt werden. Dabei wird die mit der Außenhülse verbundene Mündung auf die Außenwand der Hülse angebracht, während die mit der Innenhülse verbundene Mündung mit der Innenwand der Innenhülse verklebt wird.

Für die Bildung des Hohlraumes am Stopfen ist die Innenhülse kürzer als die Außenhülse ausgebildet, so dass der in diesem Bereich vorhandene Hohlraum für die Unterbringung des zusammengefalteten Ballons ausreicht.

In weiterer Ausgestaltung kann die Innenhülse mit einem Absperrventil in ihrem Innenraum versehen sein. Dieses kann ein Rückschlagventil sein, welches ein Fluid in der Tamponierblase zurückhält. Darüber hinaus ist es möglich, in die innere Hülse einen Kohlefilter einzusetzen, der gasdurchlässig ausgestaltet ist. Hierdurch kann eine Ableitung der entstehenden Gase erfolgen.

Das so ausgebildete Verschlußsystem ergibt einen guten Verschluß, der ein Austreten von Flüssigkeiten nach außen verhindert. Darüber hinaus werden Auffangbeutel oder dergleichen überflüssig. Für die Entnahme des Stuhls kann in sehr einfache Weise die innere Hülse aus der äußeren Hülse herausgezogen werden und der Ballon selbst durch die Öffnung der äußeren Hülse hindurchgezogen werden. Bei entsprechender Größenauslegung des Ballons kann der Ballon dann als Aufnahmebehälter für den Stuhl dienen.

Für den Fall, dass die Größe des Ballons hierfür nicht ausreicht, kann ein gesonderter größerer Auffangbehälter für den Stuhl verwendet werden, der mit einem ersten Anschlußteil an der Verschlußkappe und einem zweiten Anschlußteil an der Innenhülse angeschlossen werden kann. Über das zweite Anschlußteil kann die Innenhülse, die kraftschlüssig in die Außenhülse eingesetzt ist, aus der letzteren herausgezogen werden. Dabei nimmt sie den Ballon mit und zieht auch, nachdem der Ballon ganz durchgezogen ist, die Außenhülse aus der Verschlußkappe heraus. Hiernach kann der Stuhl sich vollständig in den Auffangbehälter entleeren.

Weitere Merkmale, Eigenschaften, Vorteile und Wirkungen auf der Basis der vorliegenden Erfindung ergeben sich anhand der folgenden Beschreibung mehrerer bevorzugter Ausführungsbeispiele der Erfindung sowie anhand der Zeichnung. Hierbei zeigt:
- Fig. 1: einen Schnitt durch eine Bauchwand mit dem Verschlußsystem im Längsschnitt während des Einsetzvorgangs in die Bauchwandöffnung;
- Fig. 2: im Schnitt das eingesetzte Verschlußsystem zu Beginn des Auffaltvorgangs des Ballons;
- Fig. 3: das Verschlußsystem mit aufgeblasenem Ballon;
- Fig. 4: das Verschlußsystem mit herausgezogener Innenhülse und Ballon;

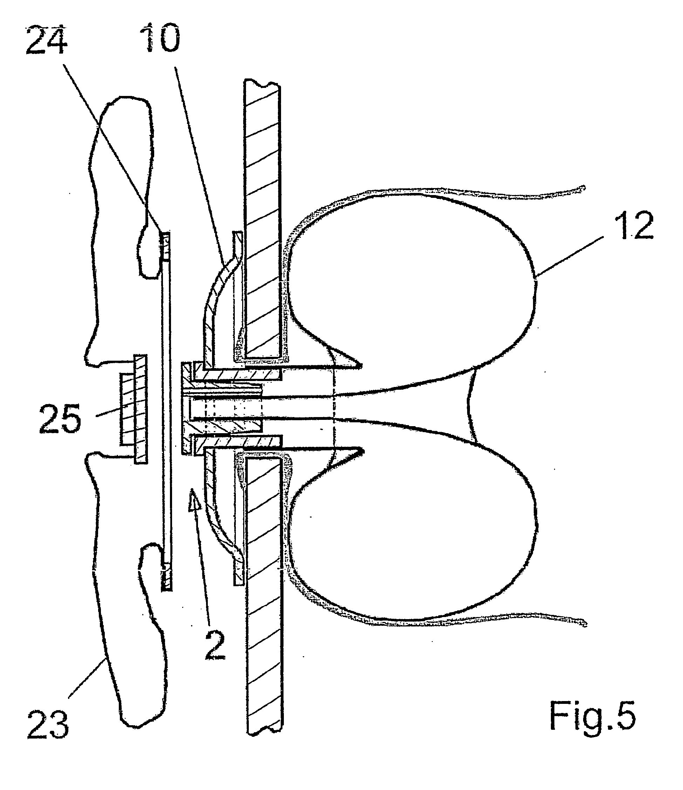

- Fig.5: das Verschlußsystem mit einem für die Anfügung daran vorbereiteten Auffangbehälter;
- Fig. 6: das Verschlußsystem mit ausgezogener Innenhülse einschließlich Ballon sowie mit dem zur Aufnahme des Stuhls vorgegebenen Auffangbehälter;
- Fig. 7: einen Schnitt durch den präforierten Ballon mit Schlauchstutzen;
- Fig.8: einen in eine dickere Bauchwandung eingesetzten Ballon mit verlängerten Schlauchstutzen;
- Fig. 9: eine dem Ballon aus Fig. 8 entsprechende Ausführungsform der Erfindung beim Einführen eines Katheters;
- Fig. 10: eine andere, für die Verwendung beim natürlichen Darmausgang optimierte Ausführungsform der Erfindung;
- Fig. 11: einen Schnitt durch die Fig. 10 entlang der Linie XI - XI;
- Fig. 12: eine der in den Fig. 10 und 11 dargestellten Ausführungsform verwandte Bauform, die sich zum Einschieben eines Drainagerohrs eignet;
- Fig. 13: eine wiederum abgewandelte Ausführungsform der Erfindung; sowie
- Fig. 14: die Verwendung der Erfindung zum Trainieren des Schließmuskels.

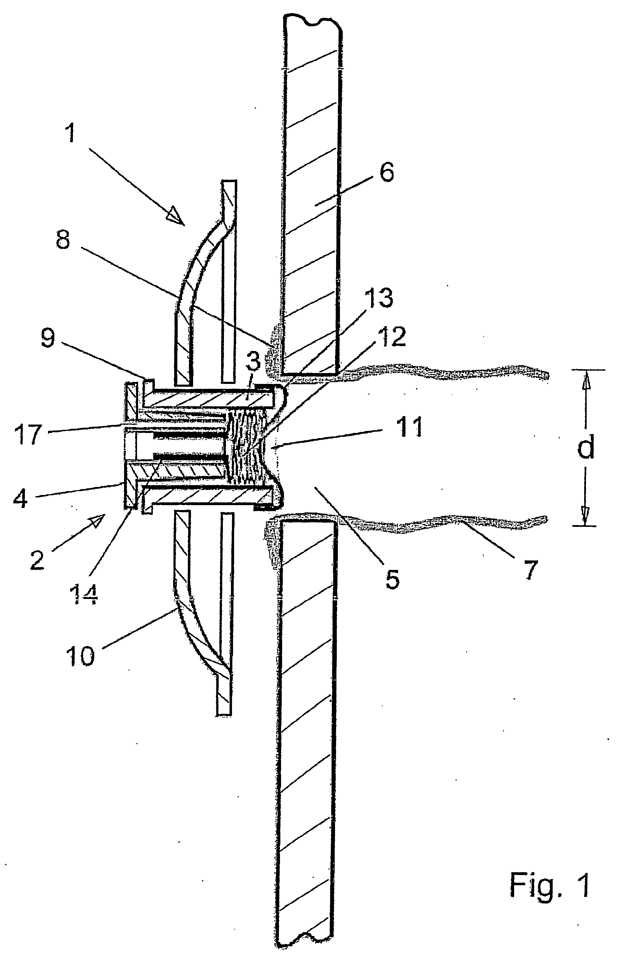

In der Fig. 1 ist das Verschlußsystem 1 für die Verwendung bei einem Anus praeter schematisch dargestellt und zwar anhand einer Ausführungsform, bei der der Stopfen 2 aus zwei ineinander steckbaren Hülsen 3 und 4 besteht. Die innere Hülse 4 ist in die Außenhülse 3 kraftschlüssig eingesteckt. Hierfür ist sie leicht konisch ausgebildet.

In der Figur ist das Verschlußsystem 1 während des Einführvorgangs in die Bauchdeckenöffnung 5 gezeigt. Die Bauchdecke 6 ist normal ausgebildet. Der Darm 7 ist mit seinem Ende 8 in an sich bekannter Weise an der Bauchdecke 6 angenäht.

Mit seinem außen liegenden Flansch 9 ergreift die Außenhülse 3 die Verschlußkappe 10, die bei vollständiger Einführung des Stopfens 2 an der Bauchdecke zur Anlage kommt und mit dieser verklebt werden kann. Es sei bemerkt, dass die Verschlußkappe 10 eine ergänzende Sicherheit für den den Patienten im Hinblick auf das Austreten von Körperflüssigkeiten bildet. Im Übrigen schützt die Kappe den nach außen auf die Körperoberfläche ausgeleiteten kurzen Darmabschnitt. Dieser ist hier ansonsten mechanischen Irritationen schutzlos ausgeliefert. Insbesondere jedoch hilft die Verschlußkappe, der hier drohenden Austrocknung und Nekrotisierung des ausgeleiteten Darmabschnittes, der ohne verhorntes Epithel, d.h. ohne natürliche Füssigkeitsbarriere vorliegt, zu vermeiden. An sich ist es auch ausreichend, wenn der Stopfen 2 beziehungsweise die Außenhülse 3 hierfür mit einem vergrößerten Ringflansch 9 versehen ist, welcher den Rand der Öffnung 5 und den ausgeleiteten Darmabschnitt überdeckt. Als Widerlager für den im Körperinneren aufgeblasenen Ballon wird die Verschlusskappe, bzw. der Flansch im Regelfall, bei erhaltender innervierung des terminalen Darmabschnittes, nicht gebraucht, da die Propulsionsbewegungen des Darmes ständig bestrebt sind, den Tamponierenden Ballon nach extrakorporal, gegen die innere Bauchwand, zu drücken.

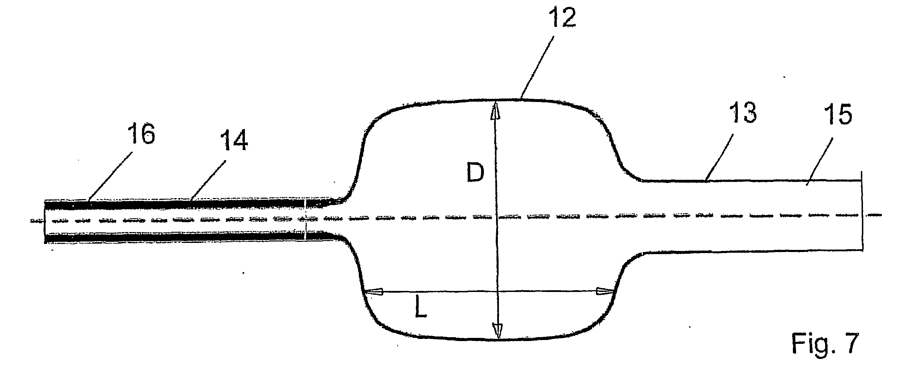

Die Innenhülse 4 ist gegenüber der Außenhülse 3 verkürzt ausgebildet, so dass ein Hohlraum 11 entsteht, in den der zusammengefaltete Ballon 12 eingefaltet werden kann. Der Ballon 12 hat, wie in der Fig. 7 gezeigt, zwei Anschlußstutzen 13 und 14, mit denen er an die Außen- beziehungsweise Innenhülse 3, 4 angeschlossen wird.

Der Ballon 12 mit den Anschlußstutzen 13 und 14 besteht aus einem dünnwandigen inflatierbaren Polymer und hat aufgeblasen einen Durchmesser D, der deutlich größer ist als der Durchmesser d des betreffenden Darmabschnitts. Der Durchmesser D wird in verschiedenen Abmessungen hergestellt und kann auf diese Weise an die Größe des Darmdurchmessers d angepaßt werden. Dieses gilt im übrigen auch für die Ausführung des Stopfens 2 beziehungsweise der Hülsen 3, 4.

Im Ausführungsbeispiel nach der Fig. 1 wird der größere Anschlußstutzen 13 mit seiner Mündung 15 auf die Außenwand der Hülse 3 aufgezogen. Die Mündung 16 des Anschlußstutzens 14 wird an der Innenwand der Innenhülse 4 befestigt. Die Befestigung kann durch Klebemittel erfolgen, möglich sind aber auch Spannringe oder dergleichen. Für den Aufblasvorgang des Ballons 12, der durch seine Befestigung am Stopfen 2 doppelwandig ausgebildet ist, ist in der Innenhülse 4 der Kanal 17 vorgesehen.

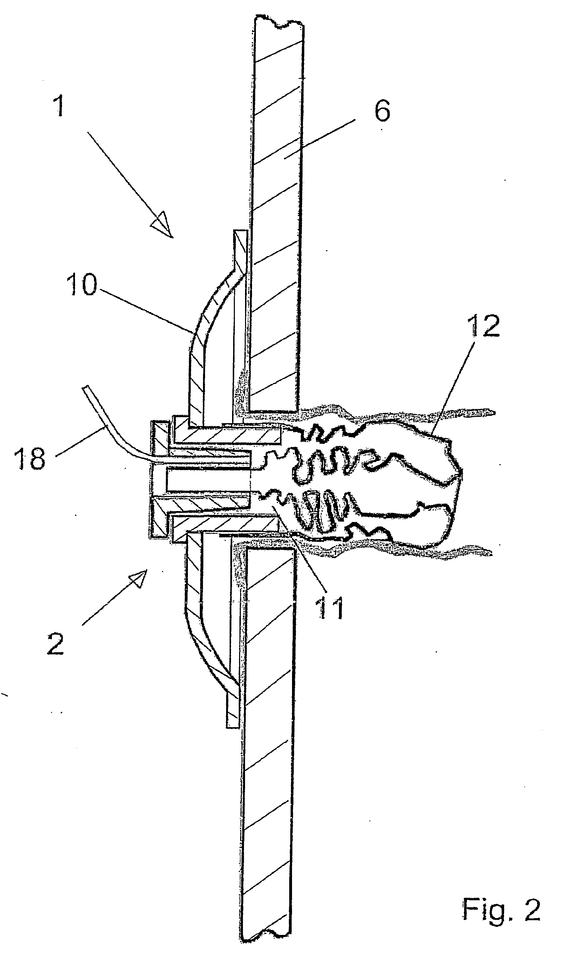

In Fig. 2 ist der Stopfen 2 voll in die Öffnung eingesetzt, so dass die Verschlußkappe 10 an der Bauchwand anliegt. Über den Schlauchnippel 18 wird Luft in den Ballon 12 eingedrückt, so dass der Ballon sich auffaltet. Der Beginn der Auffaltung ist in der Fig. 2 angegeben. Dabei ist der Ballon schon teilweise aus dem Hohlraum 11 herausgedrückt.

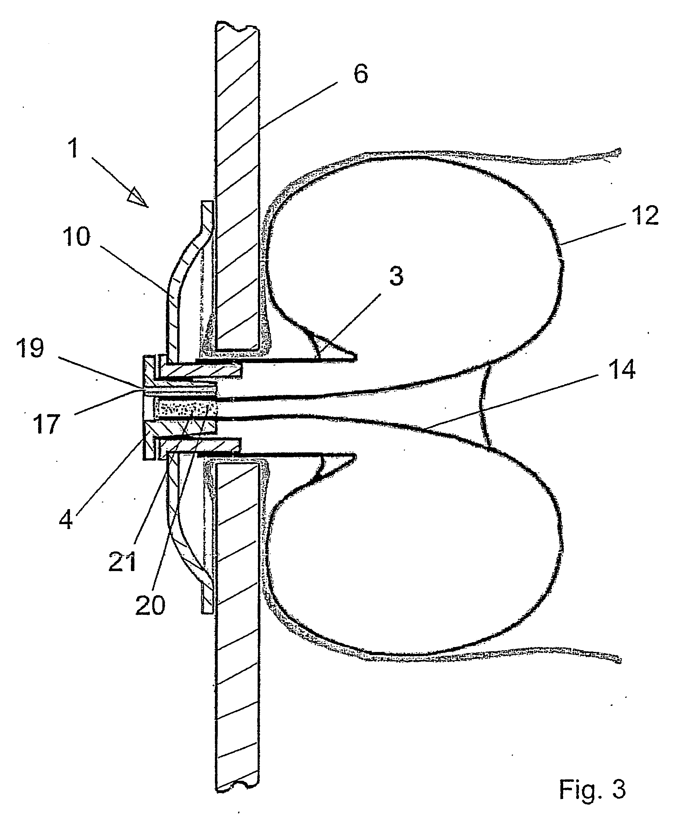

Die Fig. 3 zeigt den vollständig aufgeblasenen Ballon 12, der die Form einer Ringwulst eingenommen hat und dichtend an der Wandung des Darms 7 anliegt. Die Präformierung des Ballons 12 bei seiner Herstellung gibt seine Gestalt im aufgeblasenen Zustand wieder. Dabei kann die Ringwulst unterschiedlich lang sein, so dass sie auch walzenförmig ausgebildet ist und einen längeren Abschnitt im Darm 7 einnimmt.

Der entfaltete Ballon 12 wird in seinem Durchmesser D so ausgestaltet, dass er größer ist als der maximal gedehnte Darm, so dass sich überschüssiges Ballonwandmaterial des Außenschlauches beim Aufblasen in Falten legt, welche aufgrund der geringen Wandstärke Faltenösen in der Größenordnung von Kapillaren bilden. Flüssigkeit wird daher in den Faltenösen zurückgehalten und der extern über den Kanal 17 gemessene Druck entspricht dem auf die Darmschleimhaut ausgeübten Druck, da sich die Wandspannung des Materials nicht addiert. Der Druck auf die Darmwand 7 ist daher für eine Abdichtung ausreichend, die Gefahr einer Infarzierung des Darms wird dadurch allerdings nicht völlig vermeidbar. Günstig für die Abdichtung ist, dass die Ringwulst sich auch in Richtung der Bauchwand 6 vorwölbt und hier den Darm 7 an die Innenseite der Bauchwand 6 dichtend andrückt.

In den Luftkanal 17 der Innenhülse 4 ist ein Rückschlagventil 19 eingesetzt, welches die Luft im Ballon 12 hält. Bei Bedarf kann dieses Ventil geöffnet und die Luft abgelassen werden. Durch Anschlußstutzen 14 wird quasi eine Innenwand des Ballons 12 gebildet, die einen Austrittskanal 20 für die im Darm 7 entstehenden Gase bildet. In diesen Kanal 20 beziehungsweise in die Innenhülse 4 ist ein Kohlfilter 21 eingesetzt, der verhindert, dass flüssiger Stuhl durch den Kanal 20 austreten kann.

Um eine Entleerung des Darms vorzunehmen, ist es in vielen Fällen möglich, die Luft aus dem Ballon 12 herauszulassen beziehungsweise das Ventil 19 zu öffnen bzw. die Innenhülse 4 aus der Außenhülse 3 herauszuziehen. Das Herausziehen der Innen- aus der Außenhülse bewirkt einen Durckverlust im Tamponierballon bzw. das Deflatieren desselben. Der gesamte Ballon 12 kann dann durch den Innenraum der Außenhülse 3 hindurch gezogen werdenDer dann außen liegende Ballon 12 kann den Stuhl aufnehmen. Nach Ablösen der Kappe 10 mit der Außenhülse 3 von der Bauchdecke 6 kann der Stuhl so einfach und sicher entfernt werden.

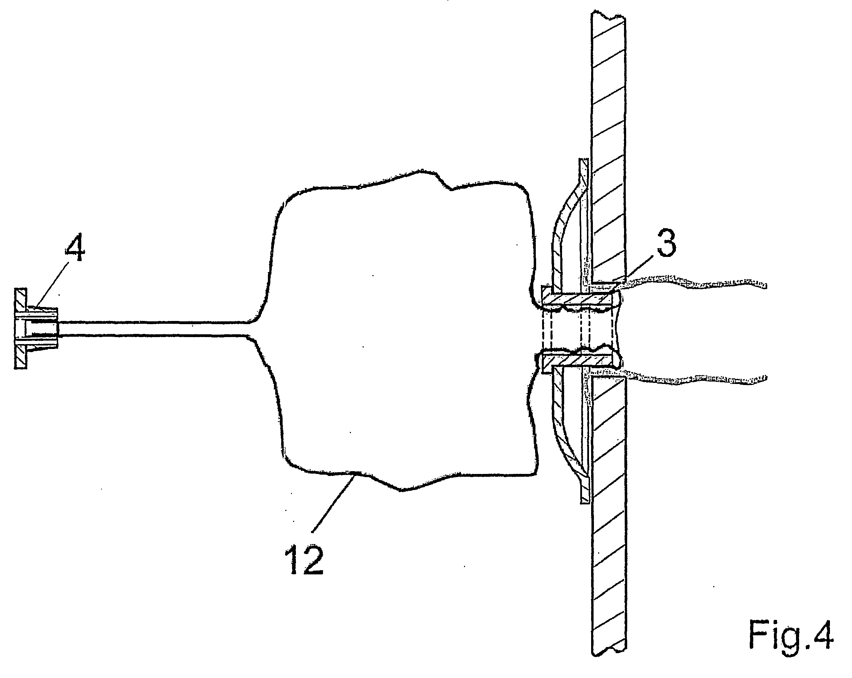

Fig. 4 zeigt die Lage des Ballons 12, in welcher dieser bereits durch die Außenhülse 3 hindurchgezogen und für die Aufnahme des Stuhls bereit ist.

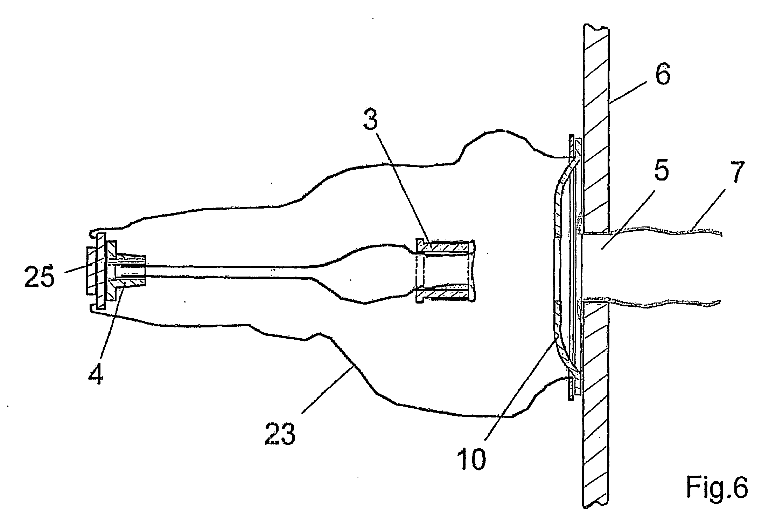

Da nicht in jedem Falle der Ballon 12 zur Aufnahme von größeren Stuhlmengen ausreicht, ist es möglich, den Stopfen 2 und/oder die Verschlußkappe 10 so auszubilden, dass an ihnen ein entsprechend ausgebildeter Auffangbeutel 23 befestigt werden kann. Der Auffangbeutel 23 hat einen an die Verschlußkappe 10 anschließbaren Ringflansch 24 sowie einen an die Innenhülse 4 aufsetzbaren Deckel 25. Durch Zug an dem Deckel 25 wird die Innenhülse 4 aus der Außenhülse 3 herausgezogen und der Ballon 12, wie bereits in den Figuren 3 und 4 geschildert, durch die innere Öffnung der Außenhülse 3 hindurchgezogen. Dieser Vorgang ist in Fig. 6 gezeigt, wobei die Außenhülse 3 ebenfalls aus der Öffnung 5 beziehungsweise seiner Halterung in der Kappe 10 herausgezogen ist, so dass der Stuhl sich in den Auffangbeutel 23 entleeren kann.

Die Fig. 7 zeigt den präformierten Ballon 12 mit den Anschlußstutzen 13 und 14. Die Anschlußstutzen 13 und 14 haben eine relativ große Länge. Vor ihrem Einsatz, das heißt, ihrer Verbindung mit dem Stopfen 2 beziehungsweise den Hülsen 3 und 4 des Stopfens 2, werden die Anschlußstutzen 13 und 14 auf eine entsprechende Länge zurecht geschnitten, je nach Dicke der Bauchdecken 6.

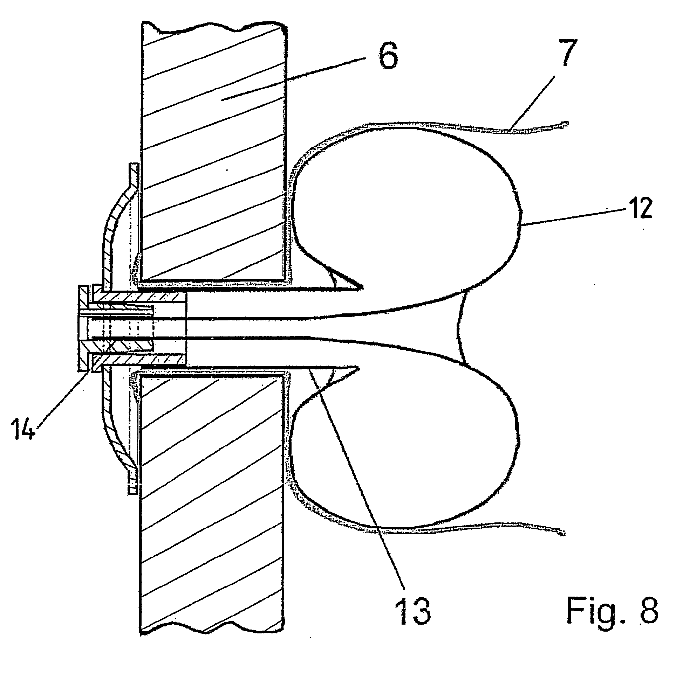

In Fig. 8 ist der Einsatz eines Ballons 12 bei stärkeren Bauchdecken 6 gezeigt, wobei alle sonstigen Teile unverändert den Teilen der Fig. 3 entsprechen. Lediglich die Anschlußstutzen 13 und 14 sind entsprechend der Bauchdecken 6 länger belassen, so dass der nicht entfaltbare Teil des Ballons 12, welchen die Anschlußstutzen 13 und 14 darstellen, weiter in den Darm 7 hineinreicht.

Besonders zu beachten ist, dass die Länge der Hülsen 3, 4 bzw. des daraus gebildete Stopfens 2 etwa der Stärke der Bauchdecken 6 entspricht bzw. nur geringfügig länger ist als diese und daher - auch bedingt durch die Tiefe der Verschlußkappe 10 - kaum in den Darm 7 hineinragt. Der weitere Verlauf des Darms 7 ist daher völlig beliebig, dieser kann auch sofort unterhalb der Bauchdecken 6 abknicken.

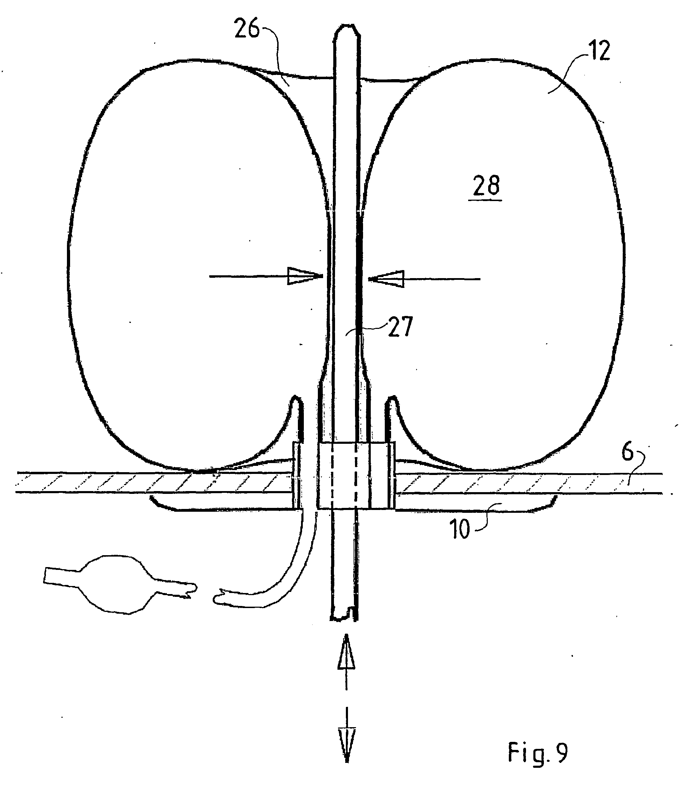

An der Anordnung gemäß Fig. 9 ist zu sehen, dass das zentrale Lumen 26 innerhalb des etwa torusförmig aufgeblasenen Ballons 12 sich insbesondere auch zum Einführen eines Katheters 27 eignet. Dabei drückt der leichte Überdruck innerhalb des Ballonvolumens 28 etwa radial nach innen gegen das zentrale Lumen 26 und klemmt den eingeführten Katheter 27 fest bzw. dichtet ihn ab. Der Katheter 27 kann im Bereich des extrakorporal gelegenen Hülsensegmentes dauerhaft fixiert, und mit einer geeigneten atraumatisch geformten Spitze sowie einer Drainageöffnung für das Ablassen von Darmgas versehen sein. Der im Verschluss fixierte Katheter ist in seiner Schaftmechanik derart ausgeführt, das er beim Einführen des Verschlusses in den Körper den auf dem Katheterschaft kollabierten Verschlussballon ohne abzuknicken trägt und so die transanale Passage des Verschlusses erleichtert. Er mißt bevorzugt nur wenige Millimeter (ca. 2-4 mm) im Durchmesser. Seine Spitze überragt das distale Ende des befüllten Ballonkörpers nur geringfügig.

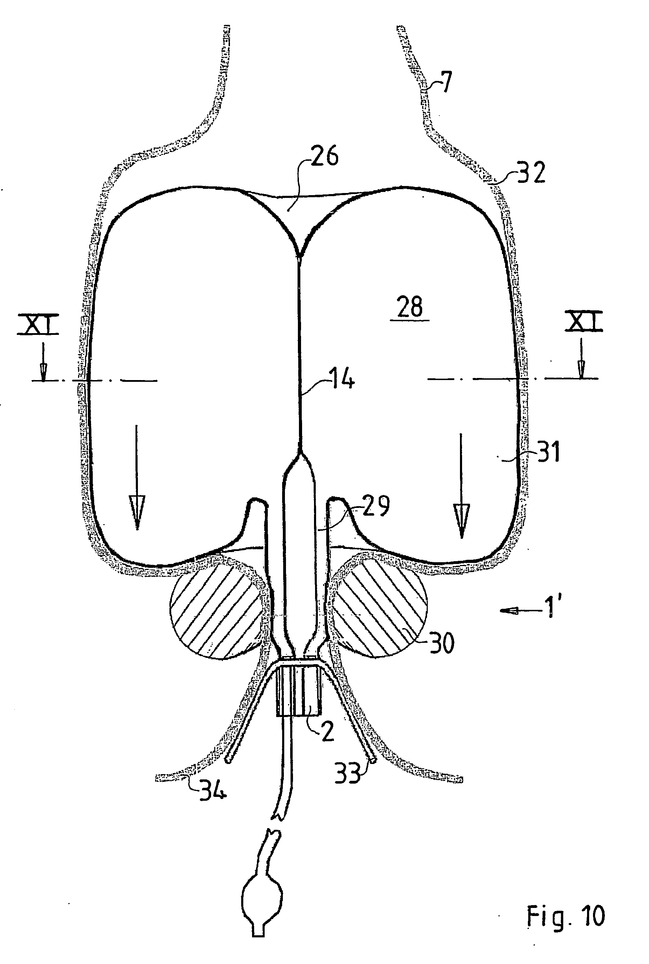

In den folgenden Figuren 10 bis 14 sind Ausführungsformen wiedergegeben, die sich zur Verwendung bei einem natürlichen Darmausgang eignen.

Das hierbei verwendete Verschlußsystem 1' unterscheidet sich von den zuvor beschriebenen nur in Details. Bspw. können die Anschlußstutzen 13, 14 etwas länger ausgebildet sein, so dass sich ein ausgeprägter Halsbereich 29 ergibt, der sich durch den Schließmuskel 30 hindurch erstreckt und es dem eigentlichen, radial erweiterten Ballonabschnitt 31 ermöglicht, die Ampulle 32 auszufüllen. Da beim Aufblähen des Ballons 12 dessen radial erweiterter Abschnitt 31 gegen den Boden der Ampulle 32 gedrückt wird, kann sich dieses Verschlußsystem 1' optimal verankern. Das Widerlager bildet dabei eine in Längsrichtung gefaltete Verschlußkappe 33, die an dem Stopfen 2 befestigt und in ihrer Gestalt an die Anatomie der Analfalte 34 angepaßt ist. Die Verschlußkappe 33 kann an den Außenseiten ihrer beiden Flügel mit einem weichen Vlies versehen sein.

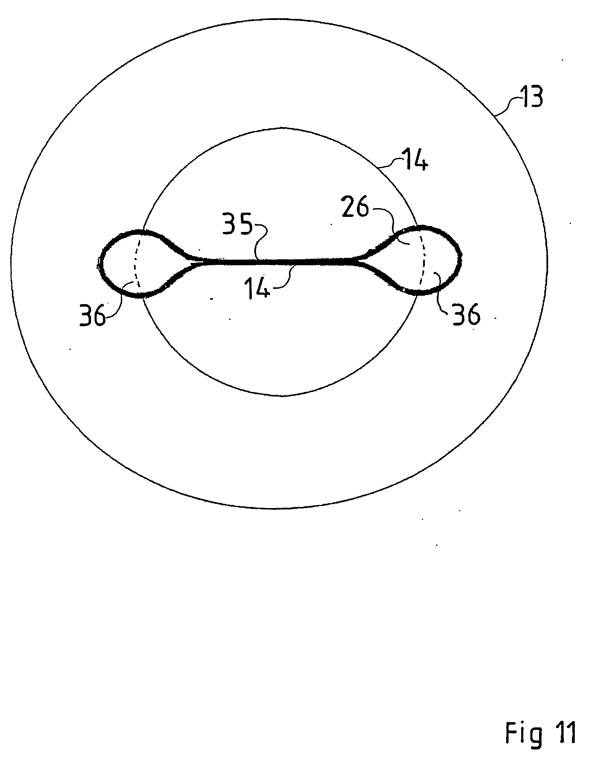

Als Nebeneffekt des Aufblähens drückt das Ballonvolumen 28 auch gegen das zentrale Lumen 26 und faltet dabei den inneren Schlauchabschnitt 14 zusammen, wie in Fig. 11 mit der dicken Linie angedeutet. Dabei wird das zentrale Lumen 26 weitgehend verschlossen. Allerdings bleiben an beiden Seiten des doppellagigen Bereichs 35 infolge der begrenzten Verformbarkeit des dickeren Schlauchmaterials am inneren Schlauchabschnitt 14 kapillarförmige Passagen 36, welche zwar den Austritt von Gasen erlauben, nicht aber von Flüssigkeiten.

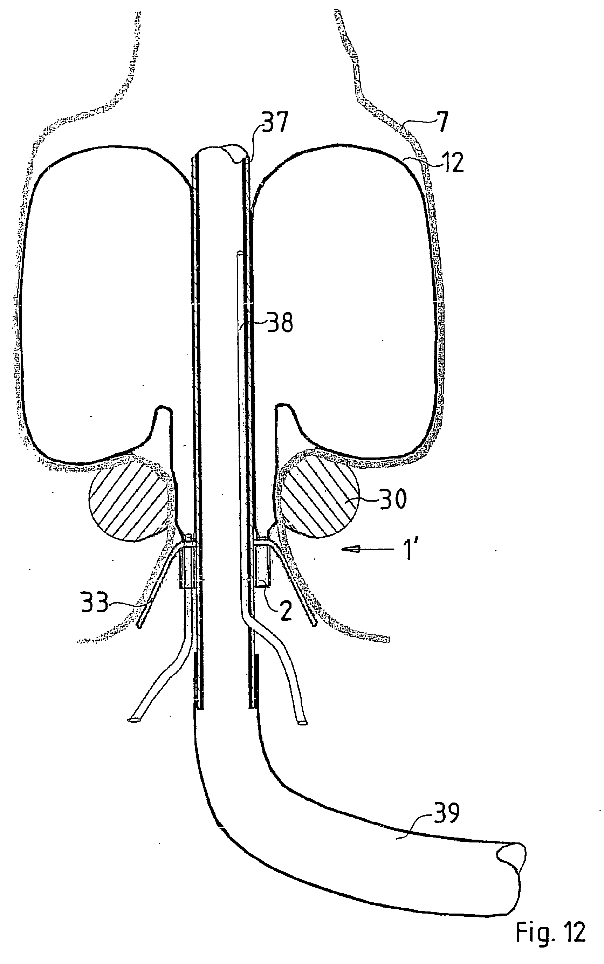

Fig. 12 veranschaulicht, dass sich diese Ausführungsform 1' auch zum Einführen eines Drainagerohrs 37 eignet, mit dem sich Spülungen des Darms 7 bewerkstelligen lassen. Hierzu wird mittels eines durch dieses Rohr 37 geführten Schlauchs 38 eine Flüssigkeit, bspw. Wasser, in den Darm 7 geleitet. Zum Ableiten der ausströmenden Flüssigkeit ist an dem externen Ende des Drainagerohrs 37 ein Schlauch 39 angeschlossen, insbesondere übergestülpt.

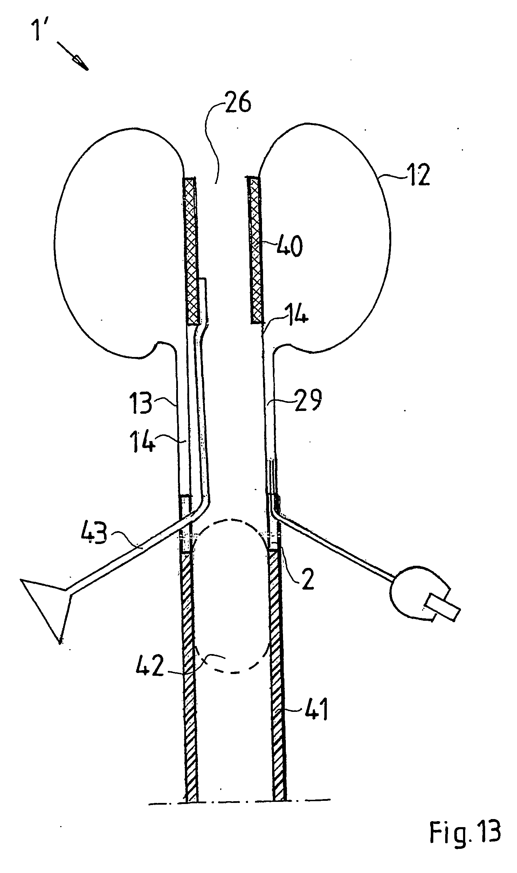

Mit dem Verschlußsystem 1' aus Fig. 13 läßt sich der natürliche Darmausgang verschließen, aber bei Bedarf auch eine Darmentleerung herbeiführen. Zu diesem Zweck wird das zentrale Lumen 26 beständig von einem Ring oder einem kurzen Rohrabschnitt 40 offengehalten, der an dem inneren Schlauchabschnitt 14 nur punkt- oder linienförmig fixiert ist. Dieser Ring oder kurze Rohrabschnitt 40 ist kürzer als die axiale Erstreckung des radial erweiterten Ballonabschnitts 31. Er ist nur über den aus den Anschlußstutzen 13, 14 gebildeten Halsabschnitt des Ballons 12 mit dem ebenfalls ringförmigen Stopfen 2 verbunden. Jenseits dieses Rings oder Stopfens 2 schließt sich ein Schlauchabschnitt 41 an. Die Ringe 2, 40 und der im Verhältnis zum Ballon 12 steifere Schlauchabschnitt 41 halten das zentrale Lumen 26 durchgehend offen, so dass eine spontane Stuhlentleerung möglich ist. Um andererseits eine solche Stuhlentleerung steuern und auch unterbinden bzw. aufschieben zu können, ist in dem zentralen Lumen 26, in dem Halsabschnitt 14, einem der Ringe 2, 40 und/oder in dem Schlauchabschnitt 41 ein beeinflußbares Verschlußelement in Form eines zweiten, getrennt aufblasbaren Ballons 42 vorgesehen, das bspw. an der Innenseite des betreffenden Abschnitts 2, 14, 26, 40, 41 mittels Klebstoff od. dgl, angeheftet sein kann und über eine getrennte Zuleitung befüllbar oder entleerbar ist. Um die Darmentleerung auch stimulieren zu können, ist eine weitere Zuleitung 43 vorgesehen, die bspw. durch den Stopfen oder Ring 2 in das zentrale Lumen 26 hinein führt und mit seinem Ende bspw. an dem vorderen Ring 40 festgelegt sein kann. Durch diese Zuleitung 43 läßt sich bspw. Wasser oder eine andere Flüssigkeit einfüllen, um den Darm zu spülen.

Wenn bei dieser Ausführungsform 1' die Länge L des bauchförmig präformierten Ballonbereichs 31 kürzer ist als dessen Außendurchmesser D, so ist - bei einem mäßigen Überdruck innerhalb des Ballons 12 - der Ring 40 zum Offenhalten des zentralen Lumens 26 nicht erforderlich, denn in diesem Fall ergibt sich eine nahezu ideale und damit im Zentrum 26 stets offene Torusform.

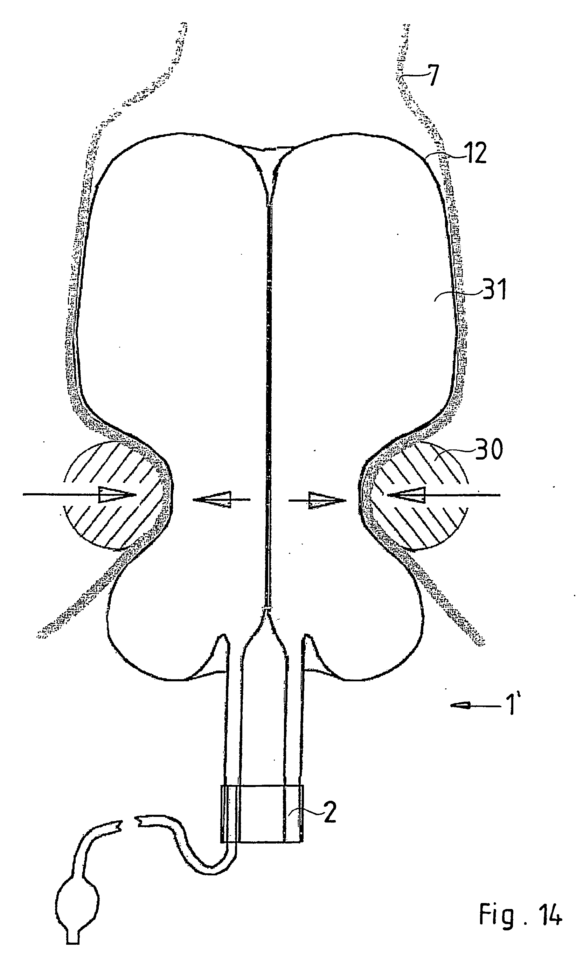

Einen anderen Anwendungsfall für das erfindungsgemäße Verschlußsystem 1' zeigt Fig. 14. Dabei wird der Ballon 12 nicht vollständig in den Darm 7 eingeführt, sondern nur teilweise, so dass er sich gerade auf Höhe des Schließmuskels 30 befindet. Nun kann durch variables Aufpumpen und Ablassen des Drucks innerhalb des Ballonvolumens 28 der Schließmuskel 30 gedehnt und damit ein entgegengesetzter Schließreflex ausgelöst werden. Durch Wiederholen dieses Vorgangs läßt sich der Schließmuskel 30 regelrecht trainieren, um dadurch Stuhlinkontinenz aktiv abzubauen. Um einer Dislokation des Ballons 12 bei normaler Körperbewegung des Patienten (Gehen, Sitzen) besser vorzubeugen, kann der Ballon im trans-analen Segment entsprechend verjüngt präformiert bzw. tailliert sein (trans-anal ca. 2 - 5 cm im Durchmesser).

Um die Verschlussvorrichtung für die Selbstversorgung bzw. -einführung von Hämorrhoidalblutungen durch den Patienten tauglich zu machen, kann der Körper durch ein Fig.9 analoges, bevorzugt dauerhaft im Hülsenabschlusssegment fixiertes, Einführ- und Drainageelement ergänzt sein.

## Patentansprüche

1. Verschlußsystem für einen natürlichen oder künstlichen Darmausgang, mit einem aufblasbaren Ballon mit einer etwa torusförmigen Struktur, **dadurch gekennzeichnet, dass** der Ballon aus einem flächigen, in sich umgestülpten Schlauchabschnitt, gebildet wird, dessen beide Enden (13,14) etwa koaxial ineinander verlaufen und mit einer oder je einer Hülse verbunden sind.

2. Verschlußsystem nach einem der vorhergehenden Ansprüche, **dadurch gekennzeichnet, dass** der Ballon (12) mit zwei Anschlußstutzen (13, 14) bzw. Enden präformiert ist.

3. Verschlußsystem nach Anspruch 1 oder 2, **dadurch gekennzeichnet, dass** die Anschlußstutzen (13, 14) bzw. Enden des Ballons (12) derart präformiert sind, dass jeder der beiden Endbereiche (13, 14) des umgestülpten Schlauchabschnitts jeweils einen etwa konstanten Querschnitt aufweist, d.h., mit einer konstanten Querschnittslänge.

4. Verschlußsystem nach einem der vorhergehenden Ansprüche, **dadurch gekennzeichnet, dass** die Anschlußstutzen (13, 14) bzw. Enden des Ballons (12) derart präformiert sind, dass Querschnitte durch die beiden Enden des umgestülpten Schlauchs unterschiedliche Längen aufweisen entsprechend unterschiedlicher Umfangslängen dieser Bereiche.

5. Verschlußsystem nach einem der vorhergehenden Ansprüche, **dadurch gekennzeichnet, dass** der umgestülpte Schlauch derart präformiert ist, dass sein vorderes, in Bezug auf die zueinander koaxialen Enden distales Ende im aufgeblähten Zustand eine sanft gewölbte Kontur annimmt ohne Kantenbereiche.

6. Verschlußsystem nach einem der vorhergehenden Ansprüche, **dadurch gekennzeichnet, dass** der Ballon derart präformiert ist, dass er im inflatierten Zustand einen über den Durchmesser d des Darmabschnitts hinausgehenden Durchmesser D aufweist.

7. Verschlußsystem nach Anspruch 6, **dadurch gekennzeichnet, dass** die Länge der Hülse oder Hülsen jeweils kleiner ist als die zur Symmetrieachse des aufgeblähten Ballons koaxiale Länge desselben, vorzugsweise kleiner als die halbe Ballonlänge, insbesondere kleiner als ein Drittel der Ballonlänge.

8. Verschlußsystem nach einem der vorhergehenden Ansprüche, **dadurch gekennzeichnet, dass** der zusammengefaltete Ballon (12) in einem in das Darminnere weisenden Hohlraum (11) eines Stopfens (2) untergebracht ist.

9. Verschlußsystem nach Anspruch 8, **dadurch gekennzeichnet, dass** zwei Anschlußstutzen (13, 14) des Ballons (12) jeweils mit ihren Mündungen an dem Stopfen (2) angeschlossen sind.

10. Verschlußsystem nach einem der Ansprüche 8 oder 9, **dadurch gekennzeichnet, dass** der Stopfen (2) aus zwei ineinander steckbaren Hülsen (3, 4) besteht und dass die Mündungen (15, 16) der Anschlußstutzen (13, 14) jeweils an eine der Hülsen (3, 4) angeschlossen sind.

11. Verschlußsystem nach einem der Ansprüche 8 bis 10, **dadurch gekennzeichnet, dass** die an die Außenhülse (3) anschließbare Mündung (15) einen an die Außenhülse (3) angepaßten Durchmesser, und die an die Innenhülse (4) anschließbare Mündung (16) einen an die Innenhülse (4) angepaßten Durchmesser haben.

12. Verschlußsystem nach einem der vorhergehenden Ansprüche, **dadurch gekennzeichnet, dass** der Ballon (12) durch eine, vorzugsweise die äußere Hülse (3) hindurchziehbar ist.

13. Verschlußsystem nach einem der vorhergehenden Ansprüche, **dadurch gekennzeichnet, dass** eine Hülse, insbesondere die Innenhülse (4), einen Luftkanal (17) aufweist.

14. Verschlußsystem nach Anspruch 13, **dadurch gekennzeichnet, dass** der Luftkanal (17) ein Absperrventil (19) aufweist.

15. Verschlußsystem nach einem der vorhergehenden Ansprüche, **dadurch gekennzeichnet, dass** im Inneren einer Hülse, vorzugsweise der Innenhülse (4), ein Filter, insbesondere ein Kohlefilter (21), angeordnet oder anordenbar ist.

16. Verschlußsystem nach einem der vorhergehenden Ansprüche, **dadurch gekennzeichnet, dass** der Stopfen (2) und/oder eine oder mehrere Hülsen mit einer Verschlußkappe (10) verbindbar ist/sind.

17. Verschlußsystem nach Anspruch 16, **dadurch gekennzeichnet, dass** die Verschlußkappe (10) mit dem Stopfen (2) formschlüssig verbunden ist.

18. Verschlußsystem nach Anspruch 16 oder 17, **dadurch gekennzeichnet, dass** die Verschlußkappe eine gefaltete Struktur aufweist.

19. Verschlußsystem nach einem der Ansprüche 16 bis 18, **dadurch gekennzeichnet, dass** die Verschlußkappe (10) und/oder der Stopfen (2) an einen Auffangbeutel (23) anschließbar ist.

20. Verschlußsystem nach einem der vorhergehenden Ansprüche 16 bis 19, **dadurch gekennzeichnet, dass** der Auffangbehälter (23) an die Verschlußkappe (10) und an die Innenhülse (4) anschließbar ist.

21. Verschlußsystem nach einem der vorhergehenden Ansprüche, **dadurch gekennzeichnet, dass** der Ballon aus einem dünnwandigen Polymer besteht.

22. Verschlußsystem nach Anspruch 21, **dadurch gekennzeichnet, dass** das Polymer ein Polyurethan, eine Polyurethan-Polyvinylchlorid-Mischung oder ein vergleichbares Material auf Polyurethan-Basis ist.

23. Verschlußsystem nach einem der vorhergehenden Ansprüche, **dadurch gekennzeichnet, dass** in dem zentralen Lumen des in sich umgestülpten Schlauchabschnitts ein ringförmiges Element fixiert ist, vorzugsweise nur entlang einer schmalen, rundumlaufenden Linie, um die Bewegungsfreiheit des Ballons nicht zu beeinträchtigen.

24. Verschlußsystem nach einem der vorhergehenden Ansprüche, **gekennzeichnet durch** ein von außen steuerbares Verschlußelement in dem zentralen Lumen des in sich umgestülpten Schlauchabschnitts oder einem daran ggf. anschließenden Schlauchabschnitt, insbesondere in Form eines getrennt aufblähbaren Ballons.

25. Verschlußsystem nach einem der vorhergehenden Ansprüche, **dadurch gekennzeichnet, dass** durch das zentrale Lumen des in sich umgestülpten Schlauchabschnitts ein Rohr, Katheter od. dgl. einführbar ist.

## Claims

1. A closing system for a natural or an artificial anus, with an inflatable balloon having an approximately toroidal structure, **characterized in that** the balloon is formed of a two-dimensional hose segment, which is inverted into itself, whereby its two ends (13,14) extend roughly coaxially inside each other and are connected to a sleeve or each to one sleeve.

2. The closing system as recited in one of the preceding claims, **characterized in that** said balloon (12) is preformed with two connection ports (13,14) or ends.

3. The closing system as recited in claim 1 or 2, **characterized in that** said connection ports (13,14) or ends of said balloon (12) are preformed such that each of the both end regions (13,14) of the inverted hose segment has a roughly constant cross section, i.e. a cross section having a constant cross-sectional length.

4. The closing system as recited in one of the preceding claims, **characterized in that** said connection ports (13,14) or ends of said balloon (12) are preformed such that cross sections through the two ends of the inverted hose have different lengths that correspond to the different circumferential lengths of said regions.

5. The closing system as recited in one of the preceding claims, **characterized in that** the inverted hose is preformed such that its front end, which is distal relative to the mutually coaxially ends, assumes in the inflated state a gently curved contour with no edge regions.

6. The closing system as recited in one of the preceding claims, **characterized in that** said balloon is preformed such that it has in the inflated state a diameter D that exceeds the diameter d of the bowel segment.

7. The closing system as recited in claim 6, **characterized in that** each length of the sleeve or sleeves is smaller than the length of the balloon measured coaxially to the axis of symmetry of the inflated balloon, preferably smaller than half the length of the balloon, particularly smaller than one-third the length of the balloon.

8. The closing system as recited in one of the preceding claims, **characterized in that** the collapsed balloon (12) is housed in a cavity (11), which is provided in a plug (2) and is directed toward the interior of the bowel.

9. The closing system as recited in claim 8, **characterized in that** two connection ports (13,14) of said balloon (12) are each connected by their mouths to said plug (2).

10. The closing system as recited in either of claims 8 or 9, **characterized in that** said plug (2) is composed of two sleeves (3,4) that can be fitted one inside the other and **in that** the mouths (15,16) of each connection port (13,14) are connected to a respective one of said sleeves (3,4).

11. The closing system as recited in one of claims 8 to 10, **characterized in that** the mouth (15) that can be connected to the outer sleeve (3) has a diameter adapted to said outer sleeve (3), and the mouth (16) that can be connected to the inner sleeve (4) has a diameter adapted to said inner sleeve (4).

12. The closing system as recited in one of the preceding claims, **characterized in that** said balloon (12) can be pulled through a sleeve, preferably through said outer sleeve (3).

13. The closing system as recited in one of the preceding claims, **characterized in that** a sleeve, particularly said inner sleeve (4), comprises an air channel (17).

14. The closing system as recited in claim 13, **characterized in that** said air channel (17) comprises a stop valve (19).

15. The closing system as recited in one of the preceding claims, **characterized in that** a filter, particularly a carbon filter (21) is or can be disposed inside a sleeve, preferably said inner sleeve (4).

16. The closing system as recited in one of the preceding claims, **characterized in that** said plug (2) and/or one or more sleeves is/are connectable to a sealing cap (10).

17. The closing system as recited in claim 16, **characterized in that** said sealing cap (10) is connected in adjacent contact to said plug (2).

18. The closing system as recited in claim 16 or 17, **characterized in that** said sealing cap has a folded structure.

19. The closing system as recited in one of claims 16 to 18, **characterized in that** said sealing cap (10) and/or plug (2) is connectable to a collection bag (23).

20. The closing system as recited in one of the preceding claims 16 to 19, **characterized in that** the collection receptacle (23) is connectable to said sealing cap (10) and to said inner sleeve (4).

21. The closing system as recited in one of the preceding claims, **characterized in that** said balloon is made of a thin-walled polymer.

22. The closing system as recited in claim 21, **characterized in that** said polymer is a polyurethane, a polyurethane/polyvinyl chloride blend or a comparable polyurethane-based material.

23. The closing system as recited in one of the preceding claims, **characterized in that** a ring-shaped element is fixed in the central lumen of the hose segment inverted into itself, said fixing preferably being effected only along a narrow, circumferentially surrounding line so as not to deteriorate the freedom of movement of the balloon.

24. The closing system as recited in one of the preceding claims, **characterized by** an externally controllable sealing element, particularly in the form of a separately inflatable balloon, disposed in the central lumen of the hose segment inverted into itself or, as applicable, a hose region adjoining thereto.

25. The closing system as recited in one of the preceding claims, **characterized in that** a tube, catheter or the like can be inserted through the central lumen of the hose segment inverted into itself.

## Revendications

1. Système d'obturation pour un anus naturel ou artificiel, comprenant un ballon gonflable doté d'une structure à peu près torique, **caractérisé en ce que** le ballon est formé d'un tronçon de boyau de grande étendue, retourné à l'intérieur de lui-même, dont les deux extrémités (13,14) sont positionnées à peu près coaxialement l'une dans l'autre et sont reliées à une douille ou chacune à une douille respective.

2. Système d'obturation selon l'une des revendications précédentes, **caractérisé en ce que** le ballon (12) est préformé avec deux embouts de raccordement (13, 14) ou extrémités.

3. Système d'obturation selon la revendication 1 ou 2, **caractérisé en ce que** les embouts de raccordement (13, 14) ou extrémités du ballon (12) sont préformés d'une manière telle, que chacune des deux parties d'extrémité (13, 14) du tronçon de boyau retourné présente une section transversale respective sensiblement constante, c'est-à-dire avec une longueur de section transversale constante.

4. Système d'obturation selon l'une des revendications précédentes, **caractérisé en ce que** les embouts de raccordement (13, 14) ou extrémités du ballon (12) sont préformés d'une manière telle, que des coupes transversales à travers les deux extrémités du boyau retourné, présentent des longueurs différentes, en correspondance avec des longueurs différentes des circonférences de ces parties.

5. Système d'obturation selon l'une des revendications précédentes, **caractérisé en ce que** le boyau retourné est préformé d'une manière telle, que son extrémité avant, distale par rapport aux extrémités coaxiales l'une à l'autre, adopte, dans l'état gonflé, un contour cintré en douceur, sans zones en arête.

6. Système d'obturation selon l'une des revendications précédentes, **caractérisé en ce que** le ballon est préformé d'une manière telle, que dans l'état gonflé, il présente un diamètre D dépassant le diamètre d du tronçon d'intestin.

7. Système d'obturation selon la revendication 6, **caractérisé en ce que** la longueur de la douille ou des douilles est respectivement inférieure à la longueur du ballon gonflé, coaxiale à l'axe de symétrie de ce dernier, de préférence inférieure à la demi-longueur du ballon, en particulier inférieure à un tiers de la longueur du ballon.

8. Système d'obturation selon l'une des revendications précédentes, **caractérisé en ce que** le ballon (12) replié est logé dans une cavité (11), donnant à l'intérieur de l'intestin, d'un bouchon (2).

9. Système d'obturation selon la revendication 8, **caractérisé en ce que** deux embouts de raccordement (13, 14) du ballon (12) sont raccordés, par leurs embouchures respectives, au bouchon (2).

10. Système d'obturation selon l'une des revendications 8 ou 9, **caractérisé en ce que** le bouchon (2) est constitué de deux douilles (3, 4) emboîtables l'une dans l'autre et **en ce que** les embouchures (15, 16) des embouts de raccordement (13, 14) sont raccordées chacune à une douille respective (3, 4).

11. Système d'obturation selon l'une des revendications 8 à 10, **caractérisé en ce que** l'embouchure (15) raccordable à la douille extérieure (3), a un diamètre adapté à la douille extérieure (3), et l'embouchure (16) raccordable à la douille intérieure (4), a un diamètre adapté à la douille intérieure (4).

12. Système d'obturation selon l'une des revendications précédentes, **caractérisé en ce que** le ballon (12) peut être tiré à travers une douille, de préférence la douille extérieure (3).

13. Système d'obturation selon l'une des revendications précédentes, **caractérisé en ce qu'**une douille, en particulier la douille intérieure (4), présente un canal d'injection d'air (17).

14. Système d'obturation selon la revendication 13, **caractérisé en ce que** le canal d'injection d'air (17) présente une valve d'arrêt (19).

15. Système d'obturation selon l'une des revendications précédentes, **caractérisé en ce qu'**à l'intérieur d'une douille, de préférence de la douille intérieure (4), est disposé ou peut être disposé un filtre, en particulier un filtre à charbon (21).

16. Système d'obturation selon l'une des revendications précédentes, **caractérisé en ce que** le bouchon (2) et/ou une ou plusieurs douilles peut/peuvent être relié(s) à un capuchon de fermeture (10).

17. Système d'obturation selon la revendication 16, **caractérisé en ce que** le capuchon de fermeture (10) est relié au bouchon (2) par un assemblage mécanique par conjugaison de formes.

18. Système d'obturation selon la revendication 16 ou 17, **caractérisé en ce que** le capuchon de fermeture présente une structure pliée.

19. Système d'obturation selon l'une des revendications 16 à 18, **caractérisé en ce que** le capuchon de fermeture (10) et/ou le bouchon (2) peut être raccordé à une poche collectrice (23).

20. Système d'obturation selon l'une des revendications précédentes 16 à 19, **caractérisé en ce que** le récipient collecteur (23) peut être raccordé au capuchon de fermeture (10) et à la douille intérieure (4).

21. Système d'obturation selon l'une des revendications précédentes, **caractérisé en ce que** le ballon est constitué d'un polymère en paroi mince.

22. Système d'obturation selon la revendication 21, **caractérisé en ce que** le polymère est un polyuréthane, un mélange de polyuréthane et de poly(chlorure de vinyle) ou une matière comparable à base de polyuréthane.

23. Système d'obturation selon l'une des revendications précédentes, **caractérisé en ce que** dans la lumière centrale du tronçon de boyau retourné à l'intérieur de lui-même, est fixé un élément annulaire, de préférence le long seulement d'une ligne étroite circonférentielle complète, pour ne pas nuire à la liberté de mouvement du ballon.

24. Système d'obturation selon l'une des revendications précédentes, **caractérisé par** un élément d'obturation manipulable de l'extérieur, dans la lumière centrale du tronçon de boyau retourné à l'intérieur de lui-même ou d'un tronçon de boyau se raccordant éventuellement à ce dernier, en particulier sous la forme d'un ballon gonflable séparément.

25. Système d'obturation selon l'une des revendications précédentes, **caractérisé en ce qu'**un tuyau, un cathéter ou élément analogue, peut être introduit à travers la lumière centrale du tronçon de boyau retourné à l'intérieur de lui-même.
